# EUROPEAN PATENT APPLICATION

(11) **EP 4 806 884 A1**
(43) Date of publication of application: **16.09.2026**
(21) Application number: 25175570.8
(22) Date of filing: 11.05.2025
(51) Int. Cl.: C07C 323/60, A61K 9/1272

(54) **SULFUR-CONTAINING COMPOUNDS FOR TRANSFECTING CELLS**

(30) Priority: 09.03.2025 EP 25162473
(71) Applicant: ScreenFect GmbH, 76344 Eggenstein-Leopoldshafen (DE)
(72) Inventor: LEVKIN, Pavel, 76344 Eggenstein-Leopoldshafen (DE); IWOHN, Michelle, 76133 Karlsruhe (DE); WIDYAYA, Vania Tanda, 76297 Stutensee (DE); MOLLA, Mijanur, 700086 Kolkata (IN)
(74) Representative: Hoppe, Georg Johannes

(57) **Abstract**

The invention refers to a compound (lipidoid) comprising a three-component structure synthesized via a one-pot reaction involving a thiolactone derivative, an amine, and an alkyl halide and its use in cell transfection.

## Description

The invention provides compounds (lipidoids) that are particularly useful for the transfection of cells. The lipoids consist of a polar headgroup, one or more non-polar tails, and a covalent linker connecting these components. Aspects of the invention are defined by the appended claims.

With the present invention, a one-pot, three-component synthesis method for lipidoids is provided, which involves the aminolysis of a thiolactone and a nucleophilic substitution.

### Description of the Invention

Transfection is a powerful technique serving as an analytical tool for investigating gene function, regulatory mechanisms, and protein activities. Due to the difficulty of naked nucleic acids traversing cellular membranes, transfection vectors are essential for effective delivery into target cells. Non-viral transfection reagents, such as liposomes - composed of aggregated cationic lipidoids - are characterized by lower cytotoxicity, better accessibility, and cost-effectiveness. However, they typically exhibit reduced transfection efficiency, especially in hard-to-transfect cell populations. Considering the limitations associated with viral transfection agents, non-viral methods, particularly lipid-based methods, have gained considerable attention recently. A prominent application of lipid nanoparticles (LNPs) is observed in messenger ribonucleic acid (mRNA) vaccine delivery. LNPs facilitate the delivery of mRNA sequences encoding the severe acute respiratory syndrome coronavirus 2 (SARS-CoV-2) spike protein to host cells. Additionally, transfection reagents can be used in various gene therapy applications, contributing to disease treatments or symptom palliations.

In a first aspect, the invention refers to a compound that is also referred to herein as a lipidoid.

Generally, such a compound (lipidoid) has a three-component structure and comprises
a) a thiolactone derivative unit, b) a functionalized primary amine unit, and c) an alkyl or alkenyl halide unit.

In certain embodiments of the invention, the compound (lipidoid) comprises or consists of a structure according to Formula I 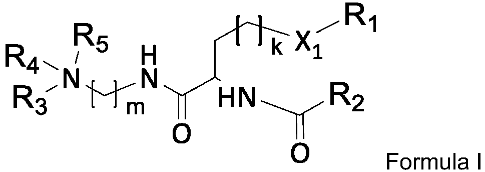 wherein
X₁ is selected from the group consisting of thioether (-S-), disulfide (-S-S-), sulfoxide (S=O) and sulfone (S(=O)₂);
R₁ is selected from the group consisting of a C6 - C24 alkyl or alkenyl, which is linear or branched; saturated or unsaturated containing 1 to 3 double bonds;
R₂ is selected from the group consisting of a C₆ - C₂₄ alkyl or alkenyl, which is linear or branched; saturated or unsaturated containing 1 to 3 double bonds;
R₃ and R₄ are the same or different and independently selected from the group consisting of:
   a C₁ - C₁₂ linear or branched alkyl (e.g., ethyl, propyl, butyl, tert-butyl); a C₁ - C₁₂ alkyl, a C₁ - C₁₂ alkenyl, or a C₁ - C₁₂ alkynyl, wherein alkyl, alkenyl or alkynyl may be optionally substituted with a C₁ - C₆ hydrocarbyl group, or R₃ and R₄ may join to form an optionally substituted heterocyclic ring of 3 to 10 atoms and 0 to 6 heteroatoms chosen from nitrogen, sulfur and oxygen;
   a hydroxyalkyl (e.g., -CH₂CH₂OH, -CH₂CH₂CH₂OH) with the length of the alkyl being between 1 and 10 (-CH₂)ₙOH; n = 1-10);
   a poly(ethylene glycol) chain (-(CH₂CH₂O)ₙH; n = 1-10);
   an oligoethylene glycol monomethyl ether (-(CH₂CH₂O)ₙCH₃; n = 1-10);
   an alkoxyalkyl (e.g., -CH₂OCH₃; generally (-CH₂)ₙOCH₃); n = 1-10); and
   any combination thereof;
   R₅ is either absent or is selected from the group consisting of a hydrogen or a C₁ - C₁₂ alkyl to provide a quaternary ammonium group;
   k is an integer from 0 to 5; and
   m is an integer from 1 to 12.

In certain embodiments of the compound, k = 1; m = 2; R₅ is absent; and/or X₁ is selected from the group consisting of thioether (-S-), and sulfoxide (S=O).

In certain embodiments, the compound comprises of consists of a structure that is selected from the group consisting of the following:

### N-(4-(decylthio)-1-((2-(diethylamino)ethyl)amino)-1-oxobutan-2-yl)tetradecanamide (T14Br10A1)

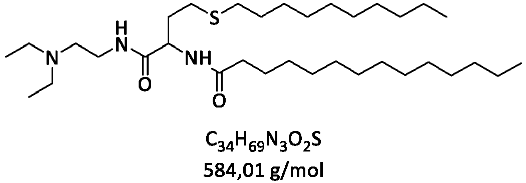

### N,N'-(3,20-diethyl-7,16-dioxo-11,12-dithia-3,6,17,20-tetraazadocosane-8,15-diyl)ditetra-decanamide ((T14A1)₂)

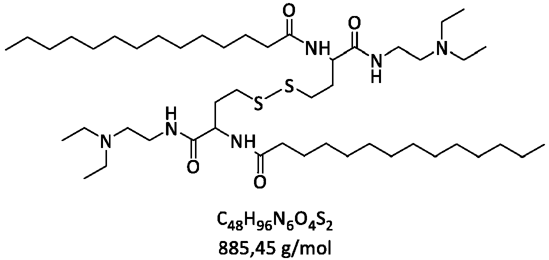

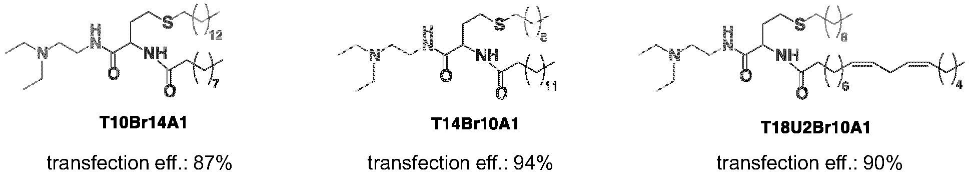

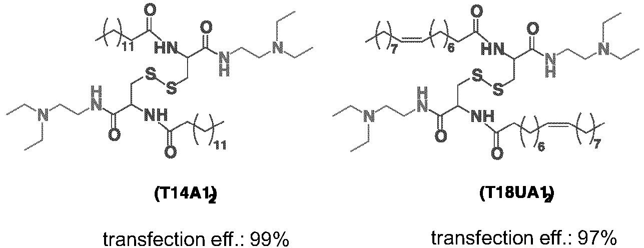

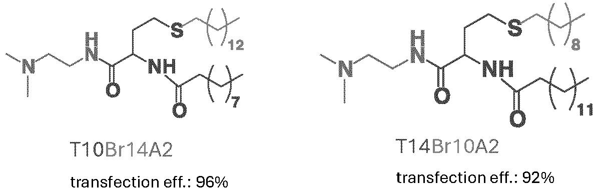

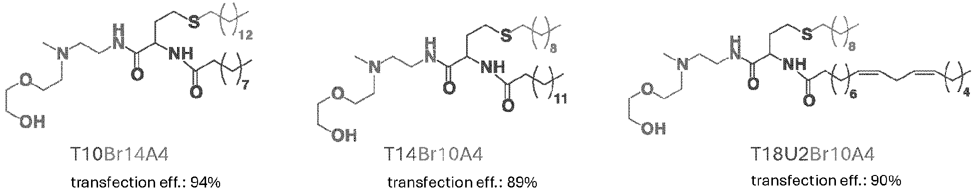

The compound comprising a three-component structure is synthesized in certain embodiments via a one-pot reaction involving the reaction of: a thiolactone derivative unit, a functionalized primary or secondary amine unit, and an alkyl or alkenyl halide unit.

This lipidoid compound is suitable for the formulation into lipid nanoparticles (LNPs) or other lipid-based transfection agents for delivering nucleic acids in vitro or in vivo.

In a second aspect, the invention refers to a method for synthesizing a lipidoid compound. In certain embodiments, the synthesis method comprises reacting with each other the following three components (units):
a) a thiolactone derivative, b) a functionalized primary amine, and c) an alkyl or alkenyl halide.

The primary amine unit is "functionalized", that is to say that it contains one or more additional functional groups (e.g. tertiary amine group, ether group, hydroxy group, ester group. etc.) linked to the primary amine through an alkyl chain.

In certain embodiments of the method, the thiolactone derivative unit is a DL-homocysteine thiolactone amide of the formula 1 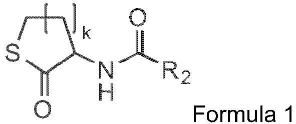 wherein
R₂ is a C₆ - C₂₄ alkyl or alkenyl, which is linear or branched; saturated or unsaturated containing 1 to 3 double bonds; and
k is a in integer from 0 to 5.

In certain embodiments of the method, the primary amine unit is of the formula 2

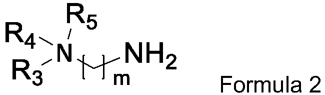

Wherein R₃ and R₄ are the same or different and independently selected from the group consisting of:
a C₁ - C₁₂ linear or branched alkyl (e.g., ethyl, propyl, butyl, tert-butyl); a C₁ - C₁₂ alkyl, a C₁ - C₁₂ alkenyl, or a C₁ - C₁₂ alkynyl, wherein alkyl, alkenyl or alkynyl may be optionally substituted with a C₁ - C₆ hydrocarbyl group, or R₃ and R₄ may join to form an optionally substituted heterocyclic ring of 3 to 10 atoms and 0 to 6 heteroatoms chosen from nitrogen, sulfur and oxygen;
a hydroxyalkyl (-(CH₂CH₂)ₙOH; with n = 1-10; e.g., -CH₂CH₂OH, or -CH₂CH₂CH₂OH);
a poly(ethylene glycol) chain (-(CH₂CH₂O)ₙH; with n = 1-10);
an oligoethylene glycol monomethyl ether (-(CH₂CH₂O)ₙCH₃; with n = 1-10);
an alkoxyalkyl (-(CH₂)ₙ-OCH₃; with n = 1-20; e.g., -CH₂OCH); and any combination thereof;
R₅ is either absent or is hydrogen or a C₁ - C₁₂ alkyl to provide a quaternary ammonium group; and m is an integer from 1 to 12.

In certain embodiments of the method, the alkyl halide unit is of Formula 3 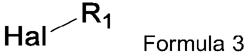
wherein Hal is a selected from the group consisting of bromide, iodide and chloride, and
R₁ is a C₆ - C₂₄ alkyl or alkenyl, which is linear or branched; saturated or unsaturated containing 1 to 3 double bonds.

In certain embodiments of the invention, the method comprises the steps of:
a) reacting the thiolactone derivative unit with a primary amine unit in the presence of an alkyl halide; and b) reacting the product of step a) with the alkyl halide to enable nucleophilic substitution of the product of step a) with an in-situ generated thiol.

In preferred embodiments, the method is a one-pot reaction resulting in steps a) and b) to occur simultaneously in the same reaction vessel.

Optionally, the method also comprises the step of preventing disulfide formation. This may be achieved by optimizing the reaction conditions. This may include adjusting the reactant ratios and degassing the reaction chamber with nitrogen. Moreover, the obtained lipidoid compound may be purified.

In certain embodiments, the reaction of the thiolactone derivative unit with the primary amine unit, in the presence of an alkyl halide is performed under aminolysis conditions.

Aminolysis conditions are reaction conditions under which disulfide formation is prevented. This may involve optimizing reaction conditions including adjusting reactant ratios, and degassing the reaction vial with nitrogen to avoid oxidation of the thiol or by adding an antioxidant reagent.

For example, the thiolactone derivative unit and the corresponding alkyl bromide can be dissolved in a solvent (e.g. ethanol or ethanol/chloroform) in a vial. The solution can be degassed, e.g. under nitrogen for, for example, 15 minutes to prevent oxidation of the in situ generated thiol. Subsequently, the amine can be added, and the mixture can be stirred at room temperature for up to 20 hours or even up to 1 week. The solvent can subsequently be removed under reduced pressure. The crude product can be dissolved in chloroform, washed sequentially with, for example, 0.1 M HCl, saturated NaHCO₃, and water, and then purified, using, for example, silica column chromatography.

In certain embodiments, the method comprises the steps a. to d. of
a. dissolving the synthesized (lipidoid) compound as described herein in an organic solvent;
b. mixing the (lipidoid) compound with a helper lipid;
c. evaporating the solvent to form a thin lipid film; and
d. forming liposomes, in particular by hydrating the film with an aqueous buffer.

In step b., the mixing of the (lipidoid) compound with a helper lipid may be doned in a molar ratio of 1:0.5 to 1:3, preferably 2:1 for a thioether lipidoid and 1:1 for a disulfide lipidoids.

In certain embodiments of the invention, the organic solvent is selected from the group consisting of ethanol, isopropanol, chloroform, dichloromethane, tetrahydrofuran, and mixtures thereof.

In certain embodiments of the invention, the helper lipid is selected from the group consisting of 1,2-dioleoyl-sn-glycero-3-phosphoethanolamine (DOPE), cholesterol, distearoylphosphatidylcholine (DSPC), dioleoylphosphatidylcholine (DOPC), and polyethylene glycol-modified phospholipids.

In certain embodiments of the invention, the thin lipid film comprises a homogeneous, continuous layer of lipidoid and helper lipid deposited on the inner surface of a vessel by solvent evaporation.

In certain embodiments of the invention, the aqueous buffer is selected from the group consisting of acetate buffer (pH 4.0-6.0), phosphate buffer (pH 6.5-7.5), HEPES buffer (pH 6.8-7.4), and Tris buffer (pH 7.0-8.0); and/or.

In certain embodiments of the method of the invention, the resulting liposomes are characterized by dynamic light scattering (DLS) and/or electrophoretic light scattering (ELS) or nanoparticle tracking analysis (NTA).

In certain embodiments of the invention, the reaction of the method is a one-pot reaction. This refers to a reaction in which all the reactants are present in the same reaction vessel.

In a third aspect, the invention refers to a lipid particle comprising a compound (lipidoid) as described above and herein.

In particular, the lipid particle is selected form the group consisting of a lipid nanoparticle (LNP), a liposome, a micelle, and a solid lipid nanoparticle, optionally in combination with one or more helper lipids.

In certain embodiments of the invention, the lipid comprises
(a) a phospholipid selected from the group consisting of phosphatidylcholine, phosphatidylethanolamine, and phosphatidylserine;
(b) a sterol selected from the group consisting of cholesterol, phytosterol, and derivatives thereof; and/or
(c) a polymer-lipid conjugate selected from the group consisting of polyethylene glycol (PEG)-lipid conjugates and polysaccharide-lipid conjugates.

In certain embodiments, the lipid further comprises a biologically active payload that is encapsulated, complexed, or adsorbed within or on the particle, wherein the payload is selected from the group consisting of plasmid DNA (pDNA), messenger RNA (mRNA), small interfering RNA (siRNA), microRNA (miRNA), antisense oligonucleotides, and proteins.

In a fourth aspect, the invention refers to a composition for delivery or for vaccination, comprising:
(a) a compound (lipidoid) as described herein;
(b) a helper lipid selected from the group consisting of 1,2-dioleoyl-sn-glycero-3-phosphoethanolamine (DOPE), cholesterol, phosphatidylcholine, dioleoylphosphatidylcholine (DOPC), distearoylphosphatidylcholine (DSPC), and polyethylene glycol-modified phospholipids;
(c) an aqueous buffer selected from acetate buffer (pH 4-6), phosphate buffer (pH 6-8), HEPES buffer (pH 6.8-7.5), or Tris buffer (pH 7-9);
(d) a nucleic acid payload as a biologically active payload selected from plasmid DNA (pDNA), messenger RNA (mRNA), small interfering RNA (siRNA), microRNA (miRNA), or antisense oligonucleotides; and
(e) optionally, one or more excipients selected from polyethylene glycol (PEG), sucrose, trehalose, or salts,
wherein the compounds and helper lipid are present in a molar ratio of 1:0.5 to 1:3 and form lipid nanoparticles encapsulating the nucleic acid payload.

In a fifth aspect, the invention refers to a pharmaceutical formulation comprising the composition for delivery or for vaccination as described above and herein.

In particular, the liposomes or lipid nanoparticles (LNPs) of the pharmaceutical formulation are prepared under sterile conditions and formulated for systemic or local administration, in particular for gene therapy applications. In certain embodiments, the pharmaceutical formulation is in the form of a vaccine.

In certain embodiments, the pharmaceutical formulation is for use in treating a genetic disorder or for vaccinating against a genetic disorder, wherein the pharmaceutical formulation delivers a nucleic acid payload. In certain embodiments, the nucleic acid payload encodes at least one therapeutic protein or at least one gene-editing component.

The genetic disorder may be selected from the group consisting of cystic fibrosis, muscular dystrophy, hemophilia, and sickle cell anemia.

In a sixth aspect, the invention refers to a non-viral gene delivery system, which comprises a compound (lipidoid) as described above and herein, formulated as liposomes. In certain embodiments of the invention, the system is adapted for *in vivo* or *in vitro* gene therapy applications.

### Examples

For the construction of a library, a total of 22 lipidoids were synthesized from 11 building blocks, differing in lipid tail length and degree of unsaturation. After assembling the lipidoids into liposomes, their transfection efficiency and cell toxicity were evaluated on HEK293T cells. Compounds were identified that achieved transfection efficiencies exceeding 85 % while maintaining high cell viability. Additionally, a hemolysis assay using human erythrocytes was conducted, with results indicating a decrease in hemolytic activity as chain length increased. This finding show that some of the lipidoids could used for in vivo applications. The library was further expanded with additional lipid tail length combinations. An optimal combination consisted of one saturated tail with 14 carbon atoms and one saturated tail with 10 carbon atoms. Furthermore, transfection efficiency was enhanced when a long saturated chain was replaced with an unsaturated chain of the same length.

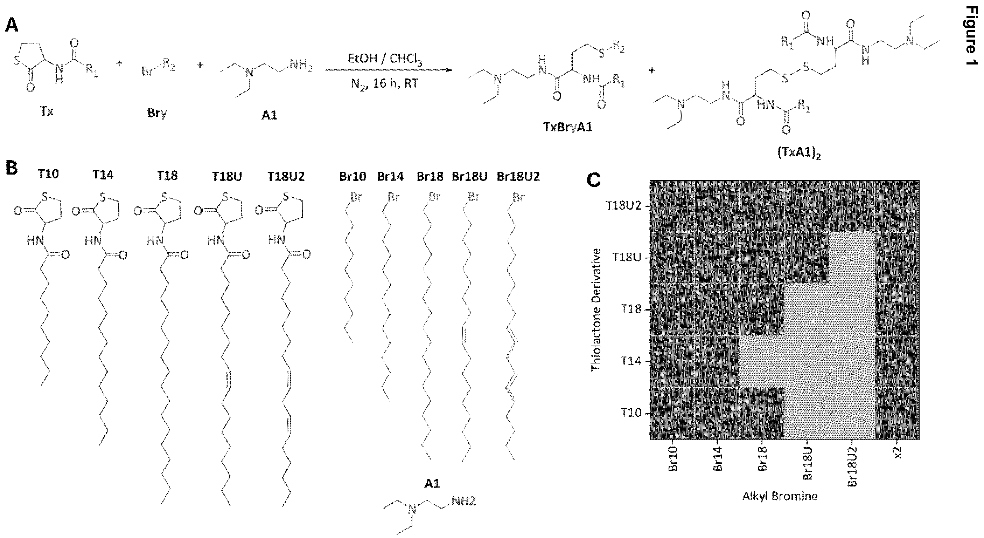

The synthesis of the compounds of the invention (lipidoids) follows a three-component, one-pot reaction, as illustrated in Figure 1A. The thiolactone derivative Tx undergoes aminolysis with the amine A1. The resulting in-situ generated thiol then attacks the alkyl bromide Bry in a nucleophilic substitution, yielding the molecule TxBryA1. Initial synthesis trials revealed the formation of a second molecule, (TxA1)₂, resulting from the oxidation of the in-situ thiol and subsequent disulfide coupling. To minimize the formation of (TxA1)₂, the synthesis conditions were optimized. Specifically, the thiolactone derivative was mixed with an excess of 2.5-3.0 equivalents of alkyl bromide to favor nucleophilic substitution over disulfide coupling. Additionally, to limit oxygen exposure and prevent the oxidation of the in-situ generated thiols, the reaction mixture was degassed with nitrogen prior to the addition of a slight excess (1.2-1.5 equivalents) of *N,N*-diethylethane-1,2-diamine (A1). Despite these adjustments, both molecules were still obtained in the synthesis. Since both compounds meet the general criteria for lipids used in transfection reagents, they were separated via column chromatography and utilized in subsequent experiments. Molecules with the structure TxBryA1 may be referred to as thioether lipidoids, while those with the structure (TxA1)₂ may be termed disulfide lipidoids. All other compounds were synthesized as described above and herein.

The building blocks for the library are depicted in Figure 1B. This study primarily focuses on modifying the tail groups in terms of their length and degree of unsaturation. Unlike other methods, the proposed synthesis allows for independent modification of both tail groups, resulting in either symmetric or asymmetric molecules. In total, 11 building blocks (5 + 5 + 1) were employed to synthesize 17 thioether lipidoids and 5 disulfide lipidoids. The specific combinations of building blocks are shown in Figure 1C. After purification, all lipidoids were characterized by NMR spectroscopy and MALDI-TOF.

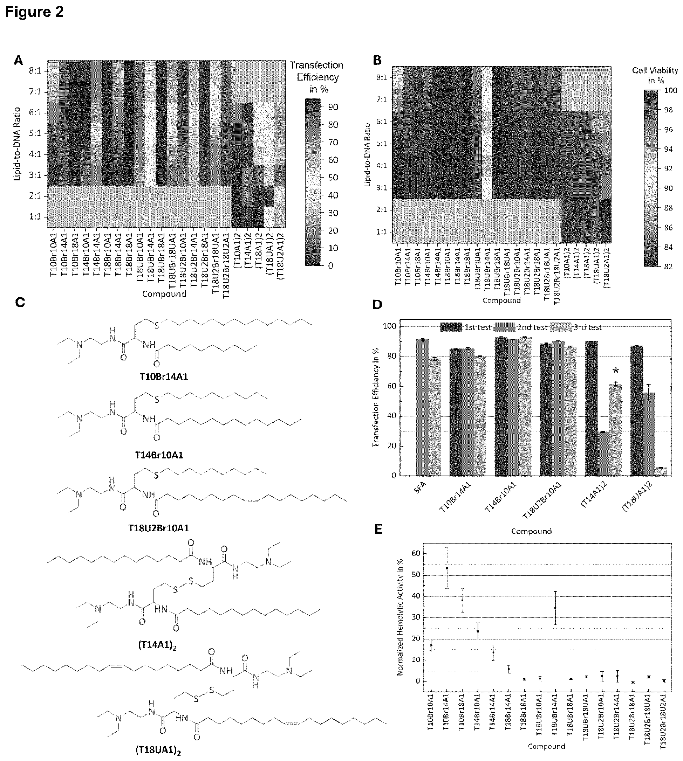

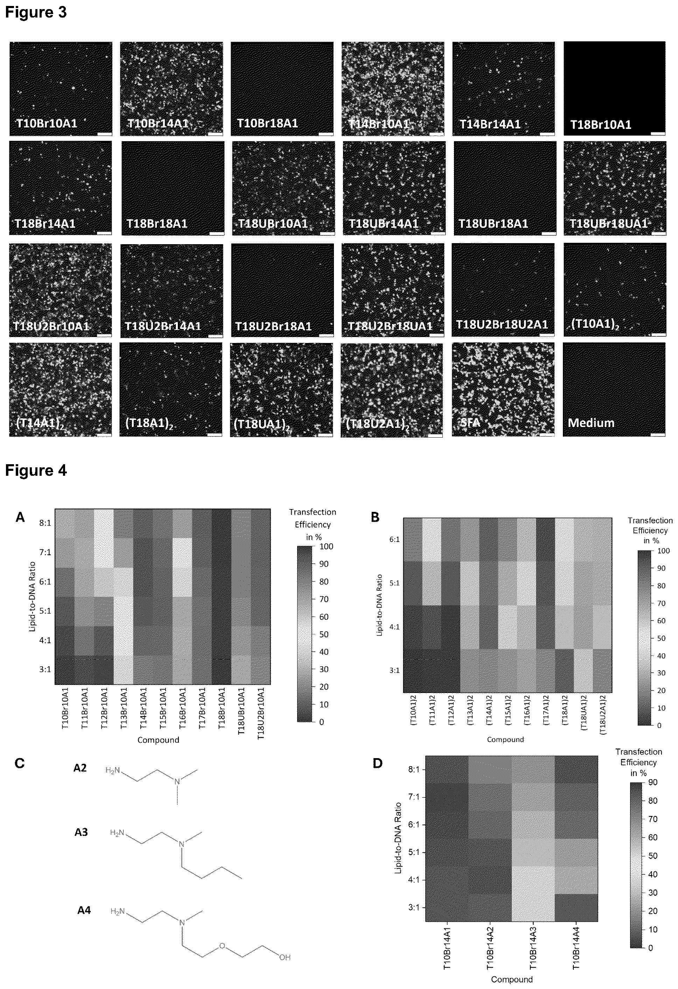

For the screening, liposomes were prepared from the synthesized lipidoids using the thin-film method in 200 mM sodium acetate buffer (pH 5.0). To enhance liposomal stability, facilitate fusion with cellular membranes, and improve the endosomal escape of the nucleic acid, 1,2-Dioleoyl-sn-glycero-3-phosphoethanolamine (DOPE) was incorporated as a helper lipid. In numerous studies, a molar ratio of 1:1 between the cationic lipid and DOPE is commonly used. To determine whether the amount of DOPE could be reduced, one compound from each structural group (T18UBr14A1 and (T18UA1)₂) was selected, and liposomes were prepared with lipidoid-to-DOPE molar ratios of 1:1 and 2:1. Both formulations were subsequently tested for transfection efficiency in HEK293T-cells across lipidoid-to-DNA ratios ranging from 1:1 to 12:1. While the general trend in transfection efficiency was consistent, significant differences in the absolute values were observed between the two lipidoid-to-DOPE ratios. For T18UBr14A1, a lipidoid-to-DOPE ratio of 2:1 consistently yielded higher transfection efficiencies across all lipidoid-to-DNA ratios. The highest transfection rate achieved was 66% for the 2:1 ratio, while the 1:1 ratio transfected only 35% of cells at the same lipidoid-to-DNA ratio. In contrast, for (T18UA1)₂, the lipidoid-to-DOPE ratio of 1:1 exhibited better performance, though the difference was less pronounced. The maximum transfection efficiency for the 1:1 composition was 87%, while the 2:1 ratio achieved only 75%. A comparison between the two compounds further highlighted the significant impact of the lipidoid-to-DNA ratio. The optimal transfection for T18UBr14A1 was observed at a lipidoid-to-DNA ratio of 4:1, whereas (T18UA1)₂ performed best at a lipidoid-to-DNA ratio of 2:1. This underscores the importance of testing liposomes across a range of lipidoid-to-DNA ratios. Based on these findings, liposomes from all thioether lipidoids were prepared using a molar lipidoid-to-DOPE ratio of 2:1, while liposomes from all disulfide lipidoids were prepared at a molar ratio of 1:1. The liposomes were characterized using dynamic light scattering (DLS) and electrophoretic light scattering (ELS). The hydrodynamic diameters ranged from 177 to 5052 nm, and the zeta potentials varied between 13 and 79 mV. Lipoplexes were formed by complexing the liposomes with plasmid DNA (pDNA) encoding the green fluorescent protein (GFP) (details in the experimental section) and were characterized using the same methods as the liposomes. In general, the lipoplexes exhibited larger hydrodynamic diameters compared to the liposomes. The zeta potentials were reduced due to the interaction with the negatively charged DNA, but they remained mostly positive. Initial in-vitro cell tests were conducted on HEK293T cells using GFP-encoding pDNA. The tests were performed in 96-well cell culture plates utilizing the one-step method. Lipoplexes were freshly prepared for each transfection by mixing 75 ng of DNA with 75-600 ng of lipidoids in the form of liposomes. Each lipidoid was tested across six different lipidoid-to-DNA ratios. Based on previous results, thioether lipidoids were tested at lipidoid-to-DNA ratios ranging from 3:1 to 8:1 (w/w), and disulfide lipidoids were tested at ratios from 1:1 to 6:1 (w/w). The lipoplexes were allowed to form for 30 minutes at room temperature before the addition of a suspension of HEK293T cells, followed by incubation for 24 hours at 37 °C. For evaluation, cells were stained with Hoechst and propidium iodide (PI) and imaged using a fluorescence microscope. Flow cytometry was performed to determine the percentage of transfected cells (transfection efficiency) and assess cell viability. A commercially available transfection agent (ScreenFect A) was used as a positive control, and several negative controls, including medium, buffer, pDNA without liposome, and liposome without pDNA, were tested on each plate. The transfection efficiencies for all lipidoids at various lipidoid-to-DNA ratios, as determined by flow cytometry, are presented in Figure 2A. The corresponding fluorescence microscope images are shown in Figure 3. In general, transfection efficiency increased with higher lipidoid-to-DNA ratios, reaching a maximum at ratios of 7:1 or 8:1 (w/w). The two unsaturated disulfide lipidoids, (T18UA1)2 and (T18U2A1)2, were exceptions to this trend, with the highest transfection efficiency observed at lipidoid-to-DNA ratios of 2:1 and 3:1 (w/w), respectively. Regarding the chemical structures of the thioether lipidoids, the results indicate that combinations of either one short (C10) saturated and one mid-length (C14) saturated chain, or one short (C10) saturated and one long (C18) unsaturated chain, yielded the best transfection efficiencies. With few exceptions, the cell viability for all lipidoids remained above 96%. In total, five lipidoids exhibited transfection efficiencies greater than 85%, and their structures are depicted in Figure 2C. To confirm the consistency and reliability of these findings, liposomes prepared from the five best performing lipidoids were tested two additional times, with a two-week interval between each experiment. The transfection efficiencies for all three tests at the optimal lipidoid-to-DNA ratio for each lipidoid are shown in Figure 2D. For the thioether lipidoids, the transfection efficiencies observed in the initial tests were consistent and stable across all three repetitions. However, the second test for the two disulfide lipidoids showed a significant decrease in transfection efficiency. To determine whether this was due to instability in the liposome formulation, a new liposome stock was prepared from the originally synthesized (T14A1)₂, which was then used for the third test. Although the transfection efficiency increased to 60%, it remained significantly lower than the initial transfection efficiency of 90%. For (T18UA1)₂, the same liposome stock was used for all repetitions, but almost no transfection was observed in the third repetition. These results indicate that neither the liposomes nor the disulfide-containing lipidoids maintain stability over extended periods. To assess the toxicity of the liposomes, their hemolytic activity was evaluated using human erythrocytes. Since the assay needed to be conducted under physiological conditions, the liposome stock solution, originally synthesized in a sodium acetate buffer at pH 5.0, was diluted with Tris HCl buffer at pH 7.6 in a 1:1 ratio. The maximum concentration of the liposome solution tested was 525 ng·µL⁻¹, with subsequent dilutions down to a concentration of 4.1 ng·µL⁻¹. Absorbance values for the positive control (0.2% Triton-X-100) were set as representing 100% hemolysis, while those for the negative control (pure buffer) were considered 0% hemolysis. Disulfide lipidoids were excluded from the experiment due to their observed instability. Figure 2E presents the hemolytic activity of the liposomes at a concentration of 62.5 ng·µL⁻¹, which corresponds to the lipidoid concentration where the best transfection results were achieved. In general, hemolytic activity decreased with increasing chain length. The lipidoid with the shortest chains, T10Br10A1, exhibited exceptionally low transfection efficiency. Except for T18UBr14A1, all lipidoids containing at least one chain with 18 carbon atoms showed hemolytic activity below 10%. For in-vivo applications, T18U2Br10A1 would be the most suitable choice, as it demonstrates high transfection efficiency (~90%) and low hemolysis (below 5%).

Among the three molecules T10Br10A1, T14Br10A1, and T18Br10A1, the lipidoid with a C14 chain demonstrates the highest transfection efficiency. A similar trend is observed for the corresponding disulfide lipidoids, (T10A1)₂, (T14A1)₂, and (T18A1)₂. To further investigate the optimal chain length and the overall trend, thioether lipidoids containing one C10 chain and one chain with 11, 12, 13, 15, 16, or 17 carbon atoms were synthesized. The corresponding disulfide lipidoids were also obtained as side products. Liposomes were prepared as described previously, and their transfection efficiency was tested. The results are shown in Figure 4. As anticipated, transfection efficiency increased with chain length, reaching a maximum for T14Br10A1 or (T14A1)₂. However, further elongation of the chain length resulted in a decline in transfection efficiency, with low transfection observed for C17 and C18 chains. Notably, the introduction of one or two double bonds to the C18 chain significantly enhanced transfection efficiency for both thioether and disulfide lipidoids. This confirms that the trend observed in the initial screening was valid and further substantiates that lipidoids containing one saturated C14 chain exhibit the highest transfection efficiency. To further analyze the structure-function-relationship, the headgroup in the molecule T10Br14A1 was modified. Instead of the already tested headgroup *N,N*-diethylethane-1,2-diamine (A1), the molecule was synthesized using N,N-dimethylethane-1,2-diamine (A2), *N*-butyl-*N*-methylethane-1,2-diamine (A3) and 2-(2-((2-aminoethyl)(methyl)amino)ethoxy)ethan-1-ol (A4). Liposomes were prepared from the synthesized lipidoids as described above and were then tested for their transfection efficiency in HEK293T cells in lipid-to-DNA ratios from 3:1 through 8:1. Compared to the lipidoid bearing the headgroup A1, the molecules synthesized with headgroups A2 and A4 lead to equally good transfection results of around 86%. However, the lipid-to-DNA ratio in which the best transfection could be achieved varied between these molecules. While the A1 headgroup achieved almost equal efficiencies over all ratios, decreasing values were observed for the headgroup A2 for ratios higher than lipid-to-DNA 4:1. The opposite trend was perceived for the headgroup A4, as the transfection efficiency increased with increasing lipid-to-DNA ratios, reaching its highest value at the highest tested lipid-to-DNA ratio of 8:1. The headgroup A3 did not perform as well as the other three headgroups, reaching a transfection efficiency of only 68%.

*Synthesis of Acyl Chlorides:* The corresponding carboxylic acid was dissolved in 20 mL dry dichloromethane. The solution was cooled to 0 °C. Oxalyl chloride was dissolved in 10 mL dry dichloromethane and added dropwise over a period of 30 minutes. The solution was stirred for 3 hours at room temperature. The solvent was evaporated under reduced pressure to obtain the pure desired product. *Synthesis of Thiolactone Derivatives:* DL-homocysteine thiolactone hydrochloride and triethylamine were dissolved in 10 mL DCM. The mixture was cooled to 0 °C. The corresponding acyl chloride was dissolved in 15 mL DCM and added dropwise to the mixture over a period of 30 minutes. The solution was stirred overnight at room temperature. The organic phase was washed with water (2 x 50 mL), saturated sodium bicarbonate solution (2 x 50 mL) and saturated sodium chloride solution (2 x 30 mL), dried over sodium sulfate and the solvent was evaporated under reduced pressure. The product was purified by column chromatography with a suitable eluent of cyclohexane and ethyl acetate. Exact preparation for every compound is given in SI. *Synthesis of Lipidoids:* Thiolactone derivative and corresponding alkyl bromide were dissolved in ethanol or a mixture of ethanol and chloroform in a crimp vial. The vial was degassed with nitrogen for 15 minutes before adding the amine. The solution was stirred overnight at room temperature. The solvent was evaporated under reduced pressure. The raw product was dissolved in 8 mL chloroform and washed with 0.1 M HCl (1 x 5 mL), saturated sodium bicarbonate solution (1 x 5 mL) and water (2 x 5 mL). The solvent was evaporated under reduced pressure. The product was purified by column chromatography with a suitable eluent of dichloromethane and methanol supplemented with triethylamine. Exact preparation for every compound is given in SI.

*Cell Culture:* Human embryonic kidney (HEK)-cells were cultured in Dulbecco's Modified Eagle Medium (DMEM) supplemented with 10% *v*/*v* fetal bovine serum (FBS) and 1% *v*/*v* penicillin-streptomycin (Pen/Strep) stock solution using a humidified incubator from the company BINDER at 37 °C under 5% CO₂ or from the company MEMMERT at 37 °C under 5% CO₂ and 96 % humidity.

*Liposome Synthesis:* 2.10 mg of a lipidoid was dissolved in 750 µL dichloromethane (anhydrous, ≥ 99.8 %) from SIGMA ALDRICH. The respective molar amount of 1,2-dioleoyl-sn-glycero-3-phosphoethanolamine (DOPE) purchased from BLDPHARM was dissolved in 250 µL ethanol absolute from VWR and sonicated for 15 minutes at 30 °C. Liposomes from thioether lipidoids were synthesized in a molar lipidoid-to-DOPE ratio of 2:1, liposomes from disulfide lipidoids in a molar ratio of 1:1. The solutions were combined in a 20 mL glass vial (28x60 mm) from VWR and the solvent was evaporated. The vial was preheated to 65 °C before adding 1 mL 200 mM sodium acetate buffer with a pH of 5.0 which was preheated to 65 °C. After vortexing for 1 minute, the vial was placed on a mechanical shaker at 750 rpm at 65 °C. After 2 hours, the liposome was obtained as a milky solution in a concentration of 2.1 mg·mL⁻¹ w.r.t. lipid. The solutions were sealed and stored at 4 °C.

*Transfection:* The transfection was done using the one-step method. Liposomes were tested in different Lipidoid-to-DNA ratios from 1:1 - 12:1 *w*/*w.* Therefore, liposome stocks were diluted according to tables x, y and z (SI) with 50 mM sodium acetate buffer (pH 5.0). 20 µL of each dilution were transferred into separate tubes before adding 20 µL GFP-pDNA-solution in 50 mM sodium acetate buffer with a concentration of 7.5 ng·µL⁻¹. The mixture was incubated for 30 minutes at room temperature for the lipoplexes to form. As a positive control, ScreenFect^{®}A in a concentration of 30 ng·µL⁻¹ was used.

*Hemolysis Assay:* The erythrocyte concentrate with blood type 0 positive was received from Städtisches Klinikum Karlsruhe. 5 mL of the blood concentrate were diluted with 45 mL 172 mM Tris HCl buffer with a pH of 7.6 at room temperature (washing buffer). The dilution was centrifuged, and the upper liquid phase was removed. This step was repeated once more. 1 mL of the liquid sediment were mixed with 10 mL washing buffer. This solution is called TK hereinafter. 172 mM Tris HCl buffer with a pH of 7.6 at 37 °C were used as the reaction buffer. 400 µL of the liposome stock solution with a concentration of 2.1 mg·mL⁻¹ were diluted with 400 µL reaction buffer to get a concentration of 1.05 mg·mL⁻¹.

The assay was performed in 96 well cell culture plates. 100 µL of reaction buffer were added to each well. For the dilution series, 100 µL of the 1.05 mg·mL⁻¹ liposome solution were mixed with the buffer in the first well. 100 µL of this solution with a concentration of 525 ng·µL⁻¹ were mixed with the buffer in the next well. This step was repeated 7 times. Therefore, the lowest concentration was 4.1 ng·µL⁻¹. 0.2% Triton-X-100 (positive control) and pure reaction buffer (negative control) were used as 100% and 0% hemolysis. 3 independent repetitions of both controls were measured on each plate. 2.5 mL of the TK solution were mixed with 47.5 mL reaction buffer and heated up to 37 °C. 100 µL of this solution was added to each well. The plate was incubated for 30 minutes at 37 °C before centrifuging the plate and moving 100 µL of the liquid phase to a flat 96 well plate. The absorbance was measured at 540 nm. The assay was performed 4 times for each liposome and concentration.

**N-(2-oxotetrahydrothiophen-3-yl)decanamide (T10):** 500 mg DL-homocysteine thiolactone hydrochloride (3.26 mmol, 1.00 Eq), 998 µL triethylamine (725 mg, 7.16 mmol, 2.20 Eq) and 709 µL decanoyl chloride (652 mg, 3.42 mmol, 1.05 Eq) were used as starting material. The reaction was performed according to the general protocol. The raw product was purified by column chromatography with an eluent of hexane and ethyl acetate (2:1). The desired product 13 was obtained as a white solid (681 mg, 2.51 mmol, 77%).

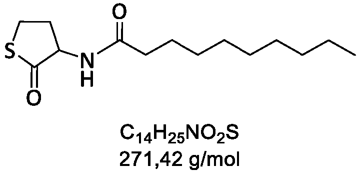

**N-(2-oxotetrahydrothiophen-3-yl)tetradecanamide (T14):** 500 mg DL-homocysteine thiolactone hydrochloride (3.26 mmol, 1.00 Eq), 998 µL triethylamine (725 mg, 7.16 mmol, 2.20 Eq) and 930 µL myristoyl chloride (844 mg, 3.42 mmol, 1.05 Eq) were used as starting material.

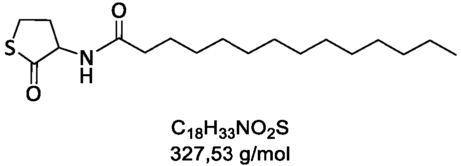

**N-(2-oxotetrahydrothiophen-3-yl)stearamide (T18):** 800 mg DL-homocysteine thiolactone hydrochloride (5.21 mmol, 1.00 Eq), 1.60 mL triethylamine (1.16 g, 11.5 mmol, 2.20 Eq) and 1.66 g stearoyl chloride (5.47 mmol, 1.05 Eq) were used as starting material.

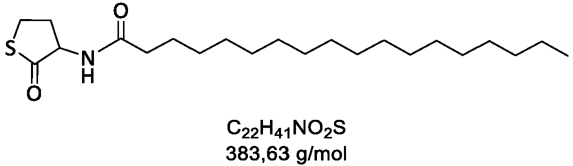

**N-(2-oxotetrahydrothiophen-3-yl)oleamide (T18U):** 800 mg DL-homocysteine thiolactone hydrochloride (5.21 mmol, 1.00 Eq), 1.60 mL triethylamine (1.16 g, 11.5 mmol, 2.20 Eq) and 1.81 mL oleoyl chloride (1.65 g, 5.47 mmol, 1.05 Eq) were used as starting material.

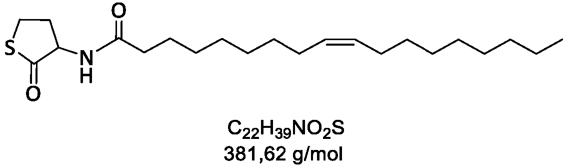

**Linoleyl chloride:** 2.44 ml linoleic acid (2.20 g, 7.84 mmol, 1.00 Eq) were dissolved in 20 mL dry dichloromethane. The solution was cooled to 0 °C. 1.49 mL oxalyl chloride (2.20 g, 17.3 mmol, 2.20 Eq) in 10 mL dry dichloromethane were added dropwise over a period of 1 h. The solution was stirred overnight at room temperature. The solvent was evaporated under reduced pressure. The desired product was obtained as a yellowish oil (2.27 g, 7.60 mmol, 97%).

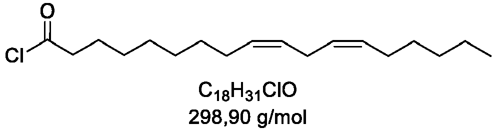

**(9Z,12Z)-N-(2-oxotetrahydrothiophen-3-yl)octadeca-9,12-dienamide (T18U2):** 800 mg DL-homocysteine thiolactone hydrochloride (5.21 mmol, 1.00 Eq), 1.60 mL triethylamine (1.16 g, 11.5 mmol, 2.20 Eq) and 1.76 mL linoleoyl chloride (1.63 g, 5.47 mmol, 1.05 Eq) were used as starting material.

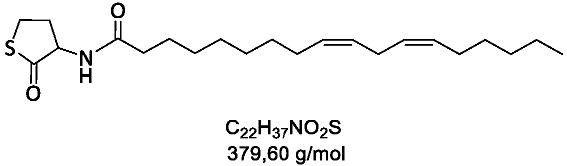

### N-(4-(decylthio)-1-((2-(diethylamino)ethyl)amino)-1-oxobutan-2-yl)decanamide (T10Br10A1)

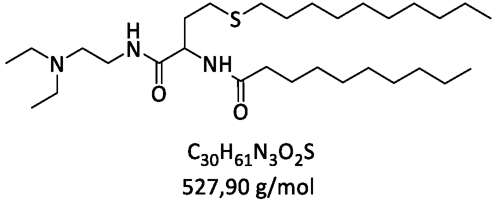

150 mg T10 (0.553 mmol, 1.00 Eq), 344 µL 1-bromodecane (367 mg, 1.66 mmol, 3.00 Eq) and 116 µL *N,N*-diethylethane-1,2-diamine (96.3 mg, 0.829 mmol, 1.50 Eq) were used as starting material.

### N-4-(tetradecylthio)-1-((2-(diethylamino)ethyl)amino)-1-oxobutan-2-yl)decanamide (T10Br14A1)

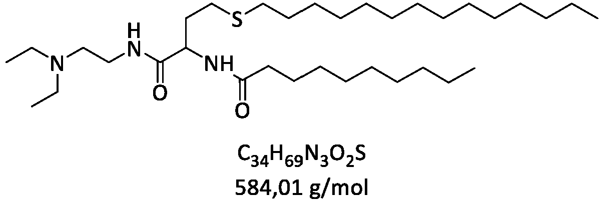

150 mg T10 (0.553 mmol, 1.00 Eq), 494 µL 1-bromotetradecane (460 mg, 1.66 mmol, 3.00 Eq) and 116 µL *N,N*-diethylethane-1,2-diamine (96.3 mg, 0.829 mmol, 1.50 Eq) were used as starting material.

### N-4-(octadecylthio)-1-((2-(diethylamino)ethyl)amino)-1-oxobutan-2-yl)decanamide (T10Br18A1)

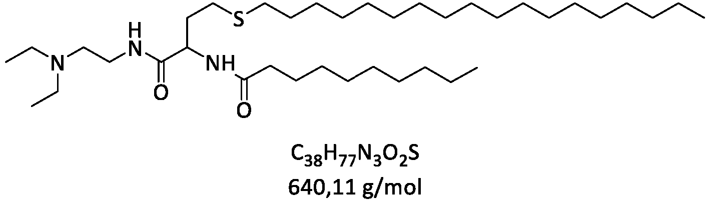

150 mg T10 (0.553 mmol, 1.00 Eq), 553 mg 1-bromooctadecane (1.66 mmol, 3.00 Eq) and 116 µL *N,N*-diethylethane-1,2-diamine (96.3 mg, 0.829 mmol, 1.50 Eq) were used as starting material.

### N-(4-(decylthio)-1-((2-(diethylamino)ethyl)amino)-1-oxobutan-2-yl)tetradecanamide (T14Br10A1)

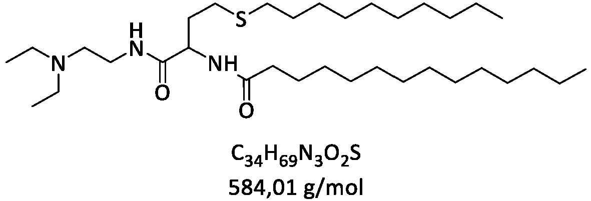

150 mg T14 (0.458 mmol, 1.00 Eq), 285 µL 1-bromodecane (304 mg, 1.37 mmol, 3.00 Eq) and 97.0 µL *N,N*-diethylethane-1,2-diamine (79.8 mg, 0.687 mmol, 1.50 Eq) were used as starting material.

### N-(4-(tetradecylthio)-1-((2-(diethylamino)ethyl)amino)-1-oxobutan-2-yl)tetradecanamide (T14Br14A1)

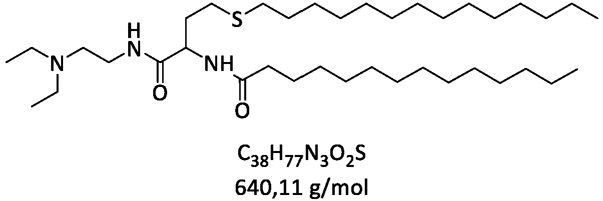

150 mg T14 (0.458 mmol, 1.00 Eq), 409 µL 1-bromotetradecane (381 mg, 1.37 mmol, 3.00 Eq) and 97.0 µL *N,N*-diethylethane-1,2-diamine (79.8 mg, 0.687 mmol, 1.50 Eq) were used as starting material.

### N-(4-(tetradecylthio)-1-((2-(diethylamino)ethyl)amino)-1-oxobutan-2-yl)stearamide (T18Br14A1)

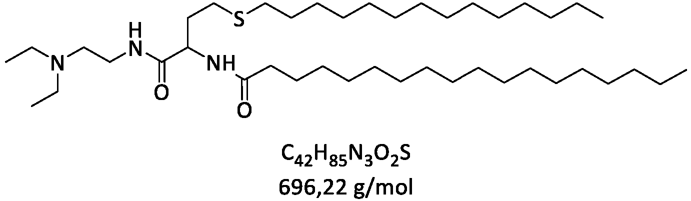

150 mg T18 (0.391 mmol, 1.00 Eq), 349 µL 1-bromotetradecane (325 mg, 1.17 mmol, 3.00 Eq) and 83.0 µL *N,N*-diethylethane-1,2-diamine (68.2 mg, 0.587 mmol, 1.50 Eq) were used as starting material.

### N-(4-(octadecylthio)-1-((2-(diethylamino)ethyl)amino)-1-oxobutan-2-yl)stearamide (T18Br18A1)

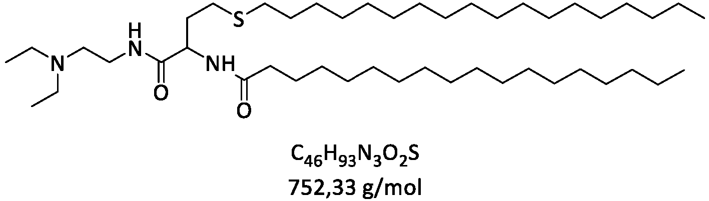

171 mg T18 (0.445 mmol, 1.00 Eq), 445 mg 1-bromooctadecane (1.34 mmol, 3.00 Eq) and 94.0 µL *N,N*-diethylethane-1,2-diamine (77.6 mg, 0.668 mmol, 1.50 Eq) were used as starting material.

### N-(4-(decylthio)-1-((2-(diethylamino)ethyl)amino)-1-oxobutan-2-yl)oleamide (T18UBr10A1)

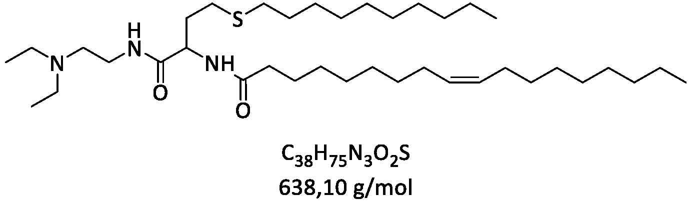

150 mg T18U (0.393 mmol, 1.00 Eq), 245 µL 1-bromodecane (261 mg, 1.18 mmol, 3.00 Eq) and 83.0 µL *N,N*-diethylethane-1,2-diamine (68.6 mg, 0.590 mmol, 1.50 Eq) were used as starting material.

### N-(4-(tetradecylthio)-1-((2-(diethylamino)ethyl)amino)-1-oxobutan-2-yl)oleamide (T18UBr14A1)

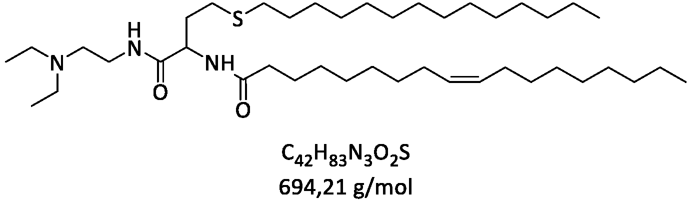

170 mg T18U (0.445 mmol, 1.00 Eq), 397 µL 1-bromotetradecane (370 mg, 1.34 mmol, 3.00 Eq) and 94.0 µL *N,N*-diethylethane-1,2-diamine (77.6 mg, 0.668 mmol, 1.50 Eq) were used as starting material.

### N-(4-(octadecylthio)-1-((2-(diethylamino)ethyl)amino)-1-oxobutan-2-yl)oleamide (T18UBr18A1)

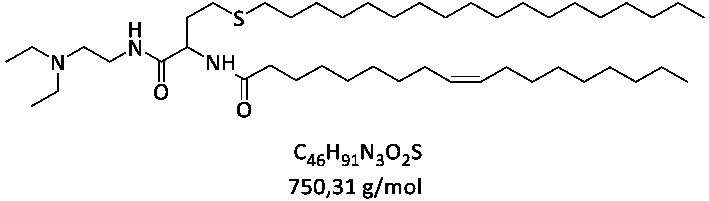

170 mg T18U (0.445 mmol, 1.00 Eq), 397 µL 1-bromotetradecane (370 mg, 1.34 mmol, 3.00 Eq) and 94.0 µL *N,N*-diethylethane-1,2-diamine (77.6 mg, 0.668 mmol, 1.50 Eq) were used as starting material.

### N-(4-(((Z)-octadec-9-en-1-yl)thio)-1-((2-(diethylamino)ethyl)amino)-1-oxobutan-2-yl)oleamide (T18UBr18UA1)

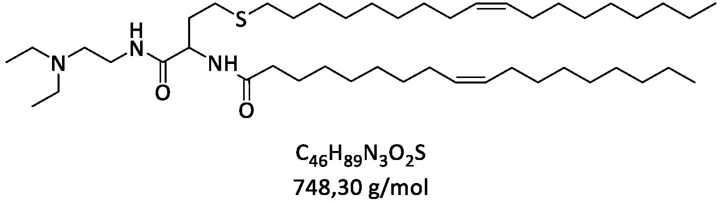

50.0 mg T18U (0.131 mmol, 1.00 Eq), 79.0 µL (Z)-1-bromooctadec-9-ene (78.5 mg, 0.236 mmol, 1.80 Eq) and 22.0 µL *N,N*-diethylethane-1,2-diamine (18.2 mg, 0.157 mmol, 1.20 Eq) were used as starting material.

### (9Z,12Z)-N-(4-(decylthio)-1-((2-(diethylamino)ethyl)amino)-1-oxobutan-2-yl)octadeca-9,12-dienamide (T18U2Br10A1)

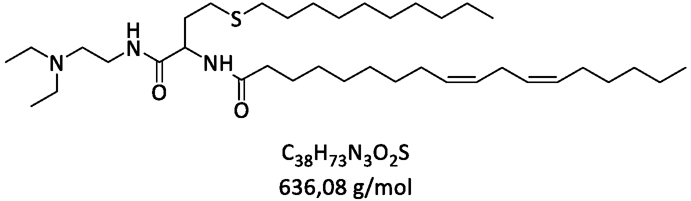

169 mg T18U2 (0.445 mmol, 1.00 Eq), 277 µL 1-bromodecane (295 mg, 1.34 mmol, 3.00 Eq) and 94.0 µL *N,N*-diethylethane-1,2-diamine (77.6 mg, 0.668 mmol, 1.50 Eq) were used as starting material.

### (9Z,12Z)-N-(4-(tetradecylthio)-1-((2-(diethylamino)ethyl)amino)-1-oxobutan-2-yl)octadeca-9,12-dienamide (T18U2Br14A1)

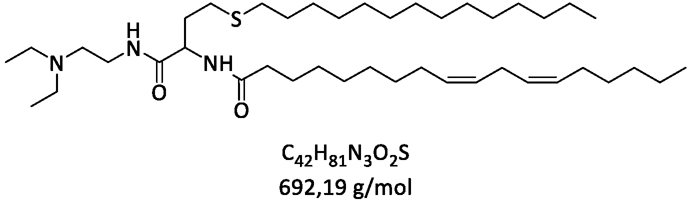

169 mg T18U2 (0.445 mmol, 1.00 Eq), 397 µL 1-bromotetradecane (370 mg, 1.34 mmol, 3.00 Eq) and 94.0 µL *N,N*-diethylethane-1,2-diamine (77.6 mg, 0.668 mmol, 1.50 Eq) were used as starting material.

### (9Z,12Z)-N-(4-(octadecylthio)-1-((2-(diethylamino)ethyl)amino)-1-oxobutan-2-yl)octadeca-9,12-dienamide (T18U2Br18A1)

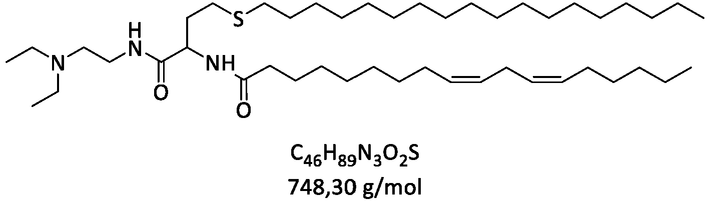

169 mg T18U2 (0.445 mmol, 1.00 Eq), 445 mg 1-bromooctadecane (1.34 mmol, 3.00 Eq) and 94.0 µL *N,N*-diethylethane-1,2-diamine (77.6 mg, 0.668 mmol, 1.50 Eq) were used as starting material.

### (9Z,12Z)-N-(4-(((Z)-octadec-9-en-1-yl)thio)-1-((2-(diethylamino)ethyl)amino)-1-oxobutan-2-yl)octadeca-9,12-dienamide (T18U2Br18UA1)

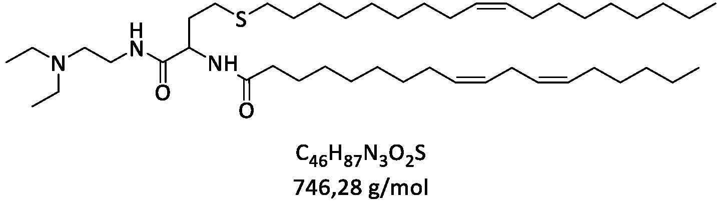

36 mg T18U2 (0.0950 mmol, 1.00 Eq), 79.0 µL (Z)-1-bromooctadec-9-ene (78.5 mg, 0.237 mmol, 2.50 Eq) and 16.0 µL *N,N*-diethylethane-1,2-diamine (13.2 mg, 0.114 mmol, 1.20 Eq) were used as starting material.

### (9Z,12Z)-N-(4-((octadeca-9,12-dien-1-yl)thio)-1-((2-(diethylamino)ethyl)amino)-1-oxobutan-2-yl)octadeca-9,12-dienamide (T18U2Br18U2A1)

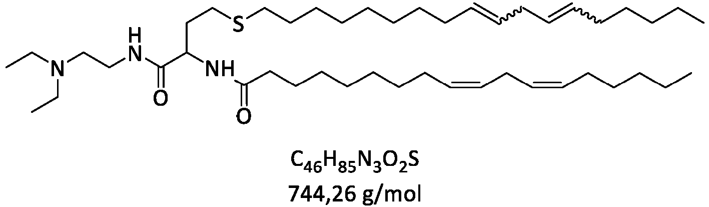

33 mg T18U2 (0.0870 mmol, 1.00 Eq), 72.3 µL 18-bromo-6,9-octadecadiene (71.9 mg, 0.218 mmol, 2.50 Eq) and 14.6 µL *N,N*-diethylethane-1,2-diamine (12.1 mg, 0.104 mmol, 1.20 Eq) were used as starting material.

### N,N'-(3,20-diethyl-7,16-dioxo-11,12-dithia-3,6,17,20-tetraazadocosane-8,15-diyl)bis(decanamide) ((T10A1)₂)

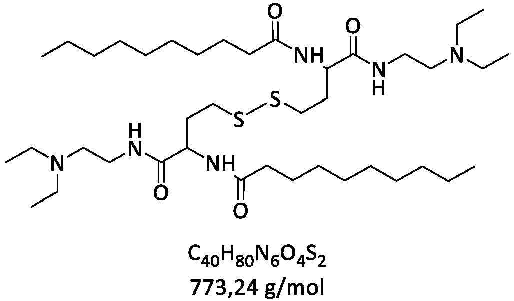

The desired product was obtained as a co-product in the synthesis of T10Br14A1 as a yellowish solid (152 mg, 0.197 mmol, 71%).

### N,N'-(3,20-diethyl-7,16-dioxo-11,12-dithia-3,6,17,20-tetraazadocosane-8,15-diyl)ditetradecanamide ((T14A1)₂)

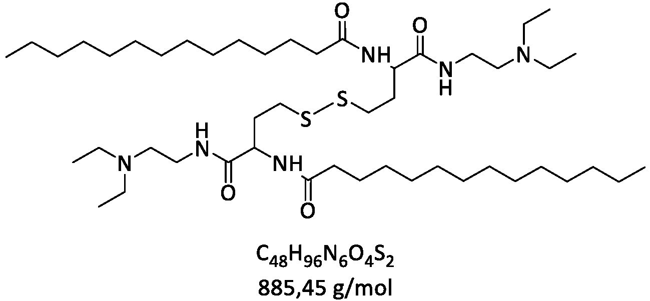

The desired product was obtained as a co-product in the synthesis of compound T14Br14A1 as a white solid (163 mg, 0.184 mmol, 80%).

### N,N'-(3,20-diethyl-7,16-dioxo-11,12-dithia-3,6,17,20-tetraazadocosane-8,15-diyl)di-stearamide ((T18A1)₂)

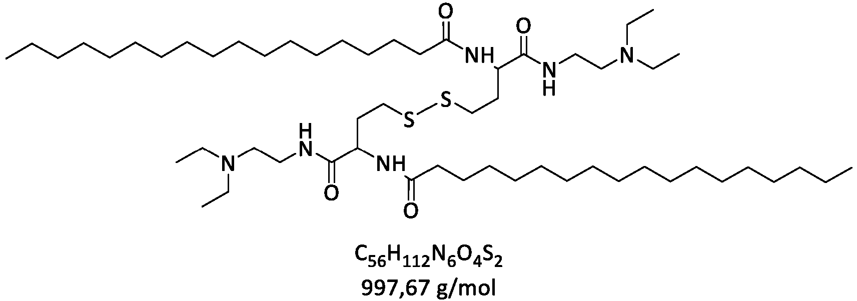

The desired product was obtained as a co-product in the synthesis of compound T18Br18A1 as a white solid (144.5 mg, 0.145 mmol, 65%).

### N,N'-(3,20-diethyl-7,16-dioxo-11,12-dithia-3,6,17,20-tetraazadocosane-8,15-diyl)di-oleamide ((T18UA1)₂)

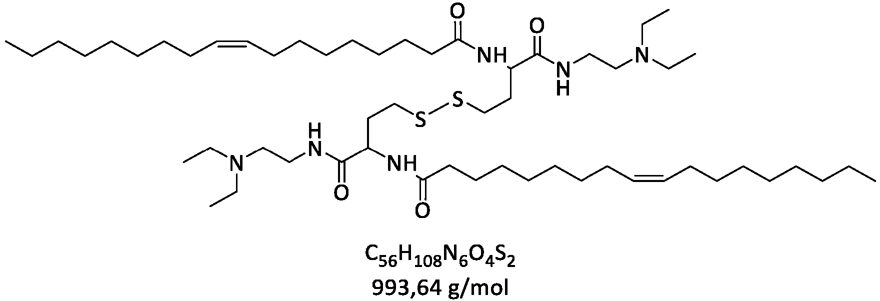

The desired product was obtained as a co-product in the synthesis of compound T18UBr14A1 as a brownish oil (207.4 mg, 0.209 mmol, 94%).

### (9Z,9'Z,12Z,12'Z)-N,N'-(3,20-diethyl-7,16-dioxo-11,12-dithia-3,6,17,20-tetraazadocosane-8,15-diyl)bis(octadeca-9,12-dienamide) ((T18U2A1)₂)

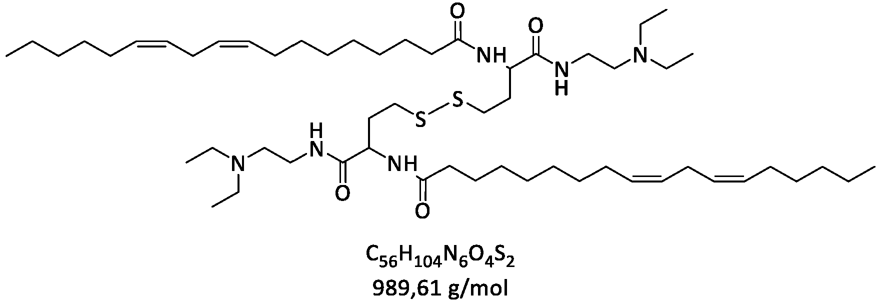

The desired product was obtained as a co-product in the synthesis of compound T18U2Br10A1 as a brownish oil (121.8 mg, 0.123 mmol, 55%).

### Undecanoyl chloride

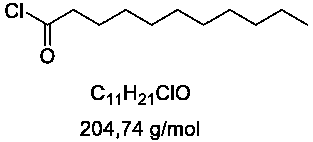

2.50 g undecanoic acid (13.4 mmol, 1.00 Eq) was dissolved in 20 mL dry dichloromethane. A solution of 1.485 mL oxalyl chloride (2.20 g, 17.3 mmol, 1.50 Eq) and 10 mL dry dichloromethane was added dropwise over a period of one hour at 0 °C. The mixture was stirred for three hours at room temperature. The solvent was evaporated under reduced pressure. The desired product was obtained as yellowish liquid (2.61 g, 12.7 mmol, 95%).

### N-(2-oxotetrahydrothiophen-3-yl)undecanamide (T11)

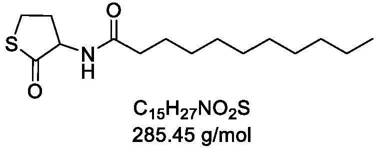

800 mg DL-homocysteine thiolactone hydrochloride (5.21 mmol, 1.00 Eq), 1.60 mL triethylamine (1.16 mg, 11.5 mmol, 2.20 Eq) and 1.21 mL undecanoyl chloride (1.12 mg, 5.47 mmol, 1.05 Eq) were used as reactants.

### N-(4-(decylthio)-1-((2-(diethylamino)ethyl)amino)-1-oxobutan-2-yl)undecanamide (T11Br10A1)

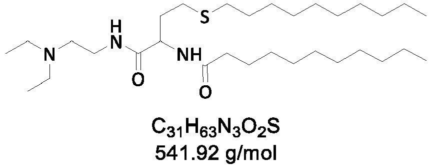

In a crimp vial, 150 mg T11 (0.525 mmol, 1.00 Eq) and 326 µL 1-bromodecane (348 mg, 1.58 mmol, 3.00 Eq) were dissolved in 5 mL ethanol. The vial was degassed with nitrogen for 15 minutes. 111 µL *N,N*-diethylethane-1,2-diamine (91.6 mg, 0.788 mmol, 1.50 Eq) was then added. The reaction mixture was stirred overnight at room temperature. The solvent was removed under reduced pressure. The raw product was dissolved in 7 mL of chloroform and washed with 5 mL of 0.1 M HCl, 5 mL of saturated sodium bicarbonate solution, and twice with 5 mL of water. The solvent was then evaporated under reduced pressure, and the product was purified by column chromatography. The eluent used was dichloromethane supplemented with 8% v/v methanol and 0.1% v/v triethylamine. The desired product was obtained as colorless solid (31.1 mg, 0.0574 mmol, 11%).

### N,N'-(3,20-diethyl-7,16-dioxo-11,12-dithia-3,6,17,20-tetraazadocosane-8,15-diyl)-diundecanamide ((T11A1)₂)

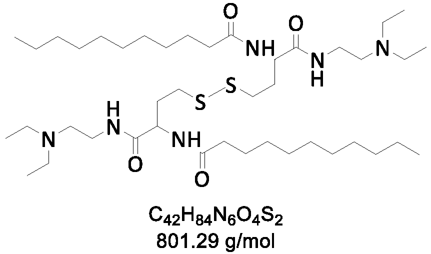

The desired product (64.0 mg, 0.0799 mmol, 46%) was isolated as a colorless solid, appearing as a co-product during the synthesis of compound T11Br10A1.

### Figures

**Figure 1****.** Synthesis of lipidoids. A) Reaction scheme of three-component one-pot reaction between a thiolactone derivative Tx, an alkyl bromide Bry and *N,N*-diethylethane-1,2-diamine (A1). The reaction results in two products TxBryA1 and (TxA1)₂. B) Structures of building blocks. Thiolactone derivatives are depicted in blue, alkyl bromides in red and *N,N*-diethylethane-1,2-diamine in green. The number in the denomination of thiolactone derivatives and alkyl bromines refers to the number of carbon atoms, U indicates an unsaturated chain and U2 a twofold unsaturated chain. C) Combinations of thiolactone derivatives and alkyl bromides used for the library. Synthesized combinations are depicted in green, compounds that could be obtained with the building blocks but were not synthesized in this work are depicted in grey.
**Figure 2****.** Transfection and toxicity tests of original library. A) Heat-map of transfection efficiencies obtained from an initial screening in HEK293T cells of all 23 lipidoids in lipidoid-to-DNA ratios between 1:1 and 8:1. B) Corresponding cell viability of HEK293T cells obtained from the same transfection tests. C) Chemical structures of the five best performing lipidoids. D) Transfection efficiencies of the five best performing lipidoids obtained from three independent tests in the best lipidoid-to-DNA ratio for each lipidoid. In test 2 and 3, all compounds were tested on the same plate and the initially prepared liposome stock solutions were used for each experiment. The interval between each test was approximately two weeks. The corresponding values for the positive control in the 2^{nd} and 3^{rd} test are shown on the left. For (T14A1)₂, the liposome was prepared freshly for the 3^{rd} test from the initially synthesized lipidoid, the bar is marked with a *. E) Hemolytic activity of liposomes from thioether lipidoids in a concentration of 62.5 ng·µL⁻¹ tested on human erythrocytes. The values are normalized to the positive control (0.2% Triton-X-100).
**Figure 3****.** Fluorescence microscope images of HEK293T-cells transfected with pDNA encoding GFP using the synthesized liposomes in 10x magnification. The positive control using a commercially available transfection agent ScreenFect A (SFA) and one negative control with medium only are shown in the lower right corner. Scale bars measure 150 µm.
**Figure 4****.** Transfection efficiencies of extended library obtained from tests in HEK293T cells. A) Heat-map showing the transfection efficiency of thioether lipidoids with the structure TxBr10A1 with x=10-18, 18U, 18U2. The lipidoids were tested in Lipidoid-to-DNA ratios of 3:1 to 8:1. B) Heat-map showing the transfection efficiency of disulfide lipidoids with the structure (TxA1)₂ with x=10-18, 18U, 18U2.The lipidoids were tested in lipidoid-to-DNA ratios of 3:1 to 6:1.

## Claims

1. Compound, comprising a structure according to Formula I 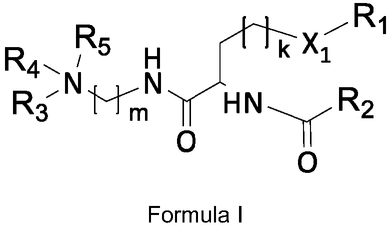 wherein
X₁ is selected from the group consisting of thioether (-S-), disulfide (-S-S-), sulfoxide (S=O) and sulfone (S(=O)₂);
R₁ is selected from the group consisting of a C6 - C24 alkyl or alkenyl, which is linear or branched; saturated or unsaturated containing 1 to 3 double bonds;
R₂ is selected from the group consisting of a C₆ - C₂₄ alkyl or alkenyl, which is linear or branched; saturated or unsaturated containing 1 to 3 double bonds;
R₃ and R₄ are the same or different and independently selected from the group consisting of:
a C₁ - C₁₂ linear or branched alkyl; a C₁ - C₁₂ alkyl, a C₁ - C₁₂ alkenyl, or a C₁ - C₁₂ alkynyl, wherein alkyl, alkenyl or alkynyl may be optionally substituted with a C₁ - C₆ hydrocarbyl group, or R₃ and R₄ may join to form an optionally substituted heterocyclic ring of 3 to 10 atoms and 0 to 6 heteroatoms chosen from nitrogen, sulfur and oxygen;
a hydroxyalkyl (-(CH₂CH₂)ₙOH; with n = 1-10);
a poly(ethylene glycol) chain (-(CH₂CH₂O)ₙH; n = 1-10);
an oligoethylene glycol monomethyl ether (-(CH₂CH₂O)ₙCH₃; n = 1-10);
an alkoxyalkyl (-(CH₂)ₙ-OCH₃; with n = 1-20);
any combination thereof;
R₅ is either absent or is selected from the group consisting of a hydrogen or a C₁ - C₁₂ alkyl to provide a quaternary ammonium group;
k is a in integer from 0 to 5; and
m is an integer from 1 to 12.

2. The compound according to claim 1, wherein
k = 1;
m = 2;
R₅ is absent; and/or
X₁ is selected from the group consisting of thioether (-S-), andsulfoxide (S=O).

3. The compound according to any of claims 1 or 2 with a structure selected from the group consisting of the following structures:
*N*-(4-(decylthio)-1-((2-(diethylamino)ethyl)amino)-1-oxobutan-2-yl)tetradecanamide (T14Br10A1) 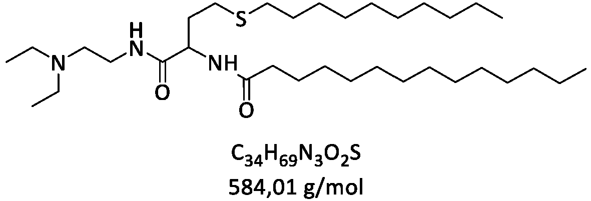
*N,N'*-(3,20-diethyl-7,16-dioxo-11,12-dithia-3,6,17,20-tetraazadocosane-8,15-diyl)ditetra-decanamide ((T14A1)₂)

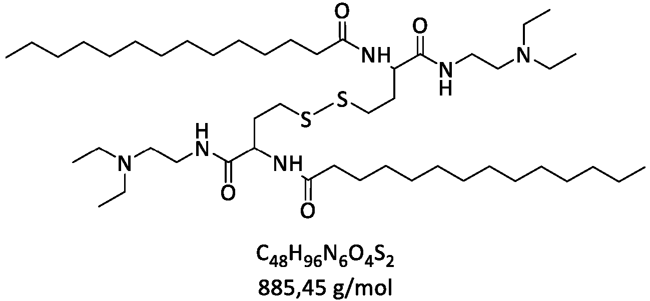

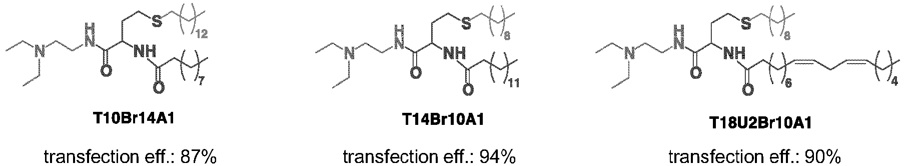

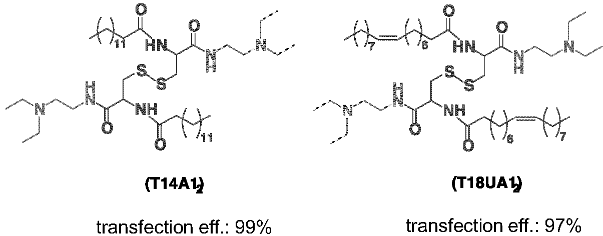

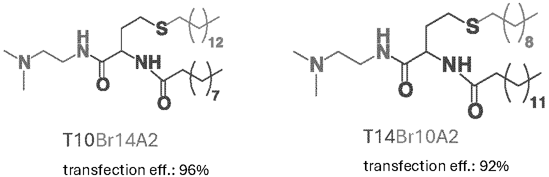

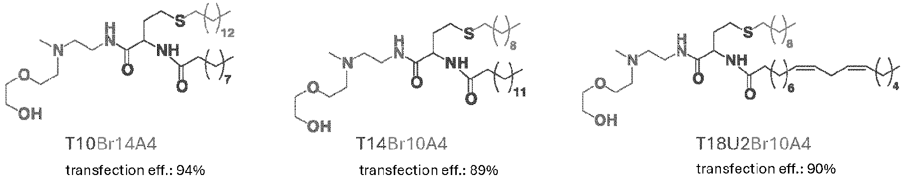

4. Method for synthesizing a lipidoid compound, in particular a compound of any of claims 1 to 3, comprising: reacting with each other
a) a thiolactone derivative unit,
b) a functionalized primary amine unit, and
c) an alkyl or alkenyl halide unit.

5. The method according to claim 4, wherein
a) the thiolactone derivative unit is a DL-homocysteine thiolactone amide of the formula 1 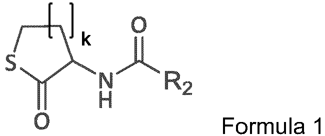 wherein
R₂ is a C₆ - C₂₄ alkyl or alkenyl, which is linear or branched; saturated or unsaturated containing 1 to 3 double bonds; and
k is a in integer from 0 to 5;
b) the primary amine unit is of the formula 2 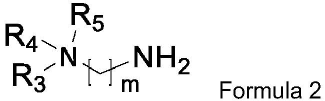 wherein
R₃ and R₄ are the same or different and independently selected from the group consisting of: a C₁ - C₁₂ linear or branched alkyl; a C₁ - C₁₂ alkyl, a C₁ - C₁₂ alkenyl, or a C₁ - C₁₂ alkynyl, wherein alkyl, alkenyl or alkynyl may be optionally substituted with a C₁ - C₆ hydrocarbyl group, or R₃ and R₄ may join to form an optionally substituted heterocyclic ring of 3 to 10 atoms and 0 to 6 heteroatoms chosen from nitrogen, sulfur and oxygen;
a hydroxyalkyl (-(CH₂CH₂)ₙOH; with n = 1-10);
a poly(ethylene glycol) chain (-(CH₂CH₂O)ₙH; with n = 1-10);
an oligoethylene glycol monomethyl ether (-(CH₂CH₂O)ₙCH₃; with n = 1-10);
an alkoxyalkyl (-(CH₂)ₙ-OCH₃; with n = 1-20); and
any combination thereof;
R₅ is either absent or is hydrogen or a C₁ - C₁₂ alkyl to provide a quaternary ammonium group; and
m is an integer from 1 to 12;
c) the alkyl halide unit is of Formula 3 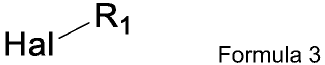
wherein Hal is a selected from the group consisting of bromide, iodide and chloride, and
R₁ is a C₆ - C₂₄ alkyl or alkenyl, which is linear or branched; saturated or unsaturated containing 1 to 3 double bonds.

6. The method of claim 4 or 5, comprising the steps of:
a) reacting the thiolactone derivative unit with a primary amine unit in the presence of an alkyl halide; and
b) reacting the product of step a) with the alkyl halide to enable nucleophilic substitution of the product of step a) with an in-situ generated thiol.

7. The method of any of claims 4 to 6, comprising the steps of:
a. dissolving the synthesized compound according to any of claims 1 to 3 in an organic solvent;
b. mixing the (lipidoid) compound with a helper lipid;
c. evaporating the solvent to form a thin lipid film; and
d. hydrating the film with an aqueous buffer to form liposomes.

8. The method of claim 7, wherein:
(a) the organic solvent is selected from the group consisting of ethanol, isopropanol, chloroform, dichloromethane, tetrahydrofuran, and mixtures thereof;
(b) the helper lipid is selected from the group consisting of 1,2-dioleoyl-sn-glycero-3-phosphoethanolamine (DOPE), cholesterol, distearoylphosphatidylcholine (DSPC), dioleoylphosphatidylcholine (DOPC), and polyethylene glycol-modified phospholipids;
(c) the thin lipid film comprises a homogeneous, continuous layer of lipidoid and helper lipid deposited on the inner surface of a vessel by solvent evaporation;
(d) the aqueous buffer is selected from the group consisting of acetate buffer (pH 4.0-6.0), phosphate buffer (pH 6.5-7.5), HEPES buffer (pH 6.8-7.4), and Tris buffer (pH 7.0-8.0); and/or
(e) optionally, characterizing the resulting liposomes by dynamic light scattering (DLS) and/or electrophoretic light scattering (ELS) or nanoparticle tracking analysis (NTA).

9. The method of any of claims 4 to 8, wherein the reaction is a one-pot reaction.

10. A lipid particle comprising a compound of any of claims 1 to 3,
wherein the particle is selected form the group consisting of a lipid nanoparticle (LNP), a liposome, a micelle, and a solid lipid nanoparticle, optionally in combination with one or more helper lipids.

11. The lipid particle of claim 10, comprising:
(a) a phospholipid selected from the group consisting of phosphatidylcholine, phosphatidylethanolamine, and phosphatidylserine;
(b) a sterol selected from the group consisting of cholesterol, phytosterol, and derivatives thereof; and
(c) a polymer-lipid conjugate selected from the group consisting of polyethylene glycol (PEG)-lipid conjugates and polysaccharide-lipid conjugates.

12. The lipid particle of claim 11, further comprising a biologically active payload that is encapsulated, complexed, or adsorbed within or on the particle, wherein the payload is selected from the group consisting of plasmid DNA (pDNA), messenger RNA (mRNA), small interfering RNA (siRNA), microRNA (miRNA), antisense oligonucleotides, and proteins.

13. A pharmaceutical formulation for use in delivery of a payload or in vaciation, comprising:
(a) a compound of any of claims 1 to 3;
(b) a helper lipid selected from the group consisting of 1,2-dioleoyl-sn-glycero-3-phosphoethanolamine (DOPE), cholesterol, phosphatidylcholine, dioleoylphosphatidylcholine (DOPC), distearoylphosphatidylcholine (DSPC), and polyethylene glycol-modified phospholipids;
(c) an aqueous buffer selected from acetate buffer (pH 4-6), phosphate buffer (pH 6-8), HEPES buffer (pH 6.8-7.5), or Tris buffer (pH 7-9);
(d) a nucleic acid cargo as a biologically active payload selected from plasmid DNA (pDNA), messenger RNA (mRNA), small interfering RNA (siRNA), microRNA (miRNA), or antisense oligonucleotides; and
(e) optionally, one or more excipients selected from polyethylene glycol (PEG), sucrose, trehalose, or salts,
wherein the compounds and helper lipid are present in a molar ratio of 1:0.5 to 1:3 and form lipid nanoparticles encapsulating the nucleic acid cargo.

14. A pharmaceutical formulation comprising the composition of claim 13, in particular wherein the liposomes or lipid nanoparticles (LNPs) are prepared under sterile conditions and formulated for systemic or local administration for gene therapy applications, in particular wherein the pharmaceutical formulation is in the form of a vaccine.

15. The pharmaceutical formulation of claim 14 for use in treating a genetic disorder or for vaccinating against a genetic disorder, wherein the pharmaceutical formulation delivers a nucleic acid encoding at least one therapeutic protein or at least one gene-editing component, and/or wherein the genetic disorder is selected from the group consisting of cystic fibrosis, muscular dystrophy, hemophilia, and sickle cell anemia.
